# NEW EUROPEAN PATENT SPECIFICATION

(11) **EP 1 858 855 B2**
(45) Date of publication and mention of the opposition decision: **03.03.2021**
(45) Mention of the grant of the patent: 19.06.2013
(21) Application number: 06723592.9
(22) Date of filing: 16.03.2006
(51) Int. Cl.: C07D 215/22

(54) **PROCESS OF MAKING CRYSTALLINE TYPE II ARIPIPRAZOLE**
VERFAHREN ZUR HERSTELLUNG VON KRISTALLINEM TYP-II-ARIPIPERAZOL
PROCEDE DE FABRICATION D UN ARIPIPRAZOLE TYPE II CRISTALLIN

(30) Priority: 17.03.2005 US 662552 P; 22.06.2005 US 692557 P
(43) Date of publication of application: 28.11.2007
(73) Proprietor: Synthon B.V., 6545 CM Nijmegen (NL)
(72) Inventor: ETTEMA, Gerrit, Jan, Bouke, NL-6641 GS Beuningen (NL); WESTHEIM, Raymond, Jozef, Hubertus, NL-5345 JT OSS (NL); KALMOUA, Faysal, NL-5348 AD Oss (NL)
(74) Representative: Mendivil Gil, Maria Dolores
(86) International application number: PCT/EP2006/002585
(87) International publication number: WO 2006/097343

(56) References cited:
- EP-A1- 1 686 117
- EP-B1- 0 367 141
- WO-A-2004/083183
- WO-A-2004/106322
- WO-A1-03/026659
- WO-A1-2005/009990
- WO-A2-2005/058835
- J. Bernstein: "Polymorphism in Molecular Crystals", , 2002, pages 110-119,
- Fabian and Kalman: "Acta Cryst. B60", , 2004, pages 547-558,
- Guillory: "Polymorphism in Pharmaceutical Solids", 1999, H.G.Britain (Ed.)
- Gu et al: Journal of Pharmaceutical Sciences, vol. 90, no. 11, November 2001 (2001-11),
- Europäisches Arzneibuch, 5. Ausg. 2005
- Huang et al: "Advanced Drug Delivery Reviews", , vol. 56, 2004,
- H G Brittain: "Profile of Drug Substances, Excipients and Related Methodology", , vol. 37, 2012, pages 1-29,
- The United States Pharmacopoeia, 2005
- Byrn, S et al: Pharmaceutical Res., vol. 12, no. 7, 1995, pages 945-954,

## Description

### BACKGROUND OF THE INVENTION

The present invention relates to processes of making a crystalline form of 7-[4-[4-(2,3-dichlorophenyl)-1-piperazinyl]butoxy]-3,4-dihydrocarbostyril, also known as aripiprazole.

Aripiprazole is a compound of the formula (1). 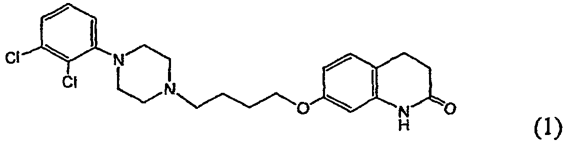 It is a commercially marketed, pharmaceutically active substance useful for treatment of schizophrenia. It is disclosed in EP 367141/US 5006528. The commercially marketed product is the free base of the title compound (1).

Solid state aripiprazole was prepared in US 5006528 by a two-fold recrystallization of crude aripiprazole from ethanol resulting in colorless flake crystals having a melting point of 139-139.5°C. In an article of Aoki (Study on Crystal Transformation of Aripiprazole, The Fourth Japan-Korea Symposium on Separation Technology, p.937 ff (1996)), this solid state form was designated as Type I aripiprazole and identified as an anhydrate. Aoki also teaches that the Type I aripiprazole may be converted into a Type II aripiprazole by heating at 130-140°C for 15 hours. This product is an anhydrate as well with a melting point of 150°C. When both Type I and Type II aripiprazole were recrystallized from an alcoholic solvent containing water up to 20%, the product is an aripiprazole hydrate labeled as Type III by Aoki. Type III aripiprazole can be converted into Type I by heating at 80°C.

WO 03/26659 (EP 1330249) teaches that the Type I aripiprazole, the alleged original solid form of aripiprazole, is significantly hygroscopic. This document also disclosed other crystalline forms of aripiprazole. One form (Form A) is a hydrate, the remaining (Forms B-G) are low hygroscopic anhydrates, differing with arrangement of molecules in the crystalline lattice.

It would be desirable to form crystalline Type II of aripiprazole without the need for a heat treatment or heat conversion. In particular, it would be desirable to find an alternate, economically more advantageous process, which does not require long-term exposure to high temperatures.

### SUMMARY OF THE INVENTION

The present invention is based on the discovery of how to form the solid state Type II aripiprazole without the use of a solid state heat treatment. Instead a liquid or solvent can be used such as by crystallizing Type II from a solvent or by a solvent-mediated solid-solid transition to form Type II in a solvent slurry.

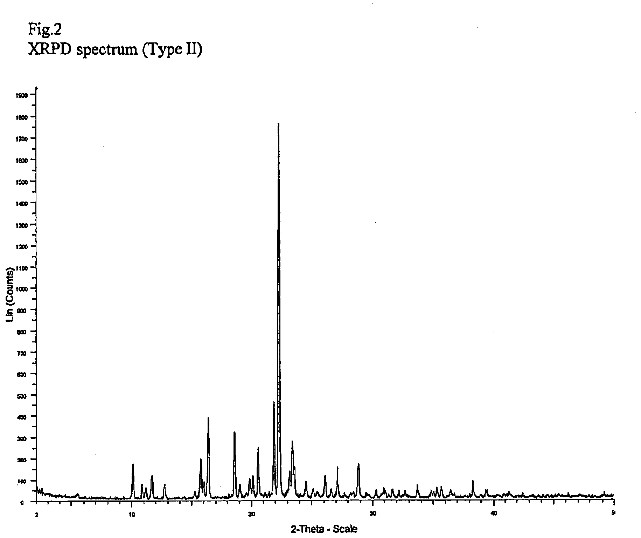

Accordingly, a first aspect of the present invention relates to a process, of making crystalline aripiprazole Type II which aripiprazole Type II having a XRPD pattern shown in Figure 2, which comprises:
a) crystallizing aripiprazole Type II from a solution of aripiprazole dissolved in a solvent selected from the group consisting of dimethyl sulfoxide, and mixtures of 2-propanol or dimethyl sulfoxide with ethyl acetate, or
b) crystallizing aripiprazole Type II from a solution of aripiprazole dissolved in a solvent consisting of 2-propanol which further comprises sieving said crystalline aripiprazole Type II to obtain a desired particle size distribution, wherein said desired particle size distribution has an average particle size of less than 200 microns, preferably less than 50 microns.

A second aspect of the invention relates to a solvent-mediated solid-solid conversion process of making aripiprazole Type II comprising stirring a suspension of aripiprazole Form B in a liquid medium, particularly in ethyl acetate, for a time sufficient to execute the solid-solid transformation from Form B to Type II.

### BRIEF DESCRIPTION OF DRAWINGS

Fig. 1 represents an example of the DSC curve of aripiprazole Type II.
Fig.2 represents an example of the XRPD pattern of aripiprazole Type II.

### DETAILED DESCRIPTION OF THE INVENTION

The invention is based on a surprising finding that aripiprazole Type II, contrary to the teaching in prior art documents, can be formed by a process, which does not require heating aripiprazole in solid state. Instead, Aripiprazole Type II may be made in a liquid environment. This easier route of formation is especially desirable because it has now been discovered that Type II aripiprazole is not hygroscopic and thus would make an excellent pharmaceutical agent from the commercialization, production, and handling perspectives.

As used herein "Type II" of aripiprazole means a crystalline aripiprazole substance having an x-ray powder diffraction (XRPD) pattern that substantially corresponds to that of the Type II product as defmed in the above cited article of Aoki. "Substantially corresponds" is meant to cover variations/differences in the pattern that would not be understood by a worker skilled in the art to represent a difference in crystal structure, but rather differences in technique, sample preparation, impurities, etc. An example of the XRPD of the Type II aripiprazole is shown on Fig. 2.

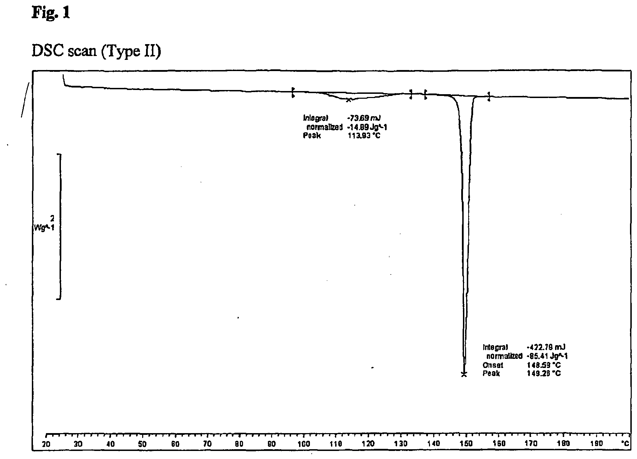

Typically the Type II aripiprazole will also have a single melting endotherm peak within the range of 145°C to 150°C, especially around 148-149°C, measured using differential thermal analysis (DTA) or differential scanning calorimetry (DSC). An example of the DSC pattern of aripirazole Type II is shown on Fig. 1. The DTA and DSC values should be used with a certain care as these types of data are dependant on measuring conditions such as heating rate, type of equipment, sample purity, sample loading, etc.

The Type II aripiprazole is a relatively stable crystalline form suitable for making pharmaceutical compositions on an industrial scale. The Type II aripiprazole (in pure state, i.e. free from other forms) is anhydrous, meaning it neither contains water or other solvent bound as part of the crystal lattice. This should be distinguished from wet crystals that have water or solvent adhered thereto. Such liquid is permitted (e.g., a "wet" or damp crystalline substance), so long as it is not part of the regular repeating unit of the crystal lattice. Generally, the Type II aripiprazole is non-hygroscopic. However, it can be hygroscopic if, *inter* alia, it is milled as discussed hereinafter.

The first process is crystallization of aripiprazole from a solvent. Such a process provides several advantages over heating aripiprazole in a solid state. The process is easily scaleable, well controllable and does not require thermal treatment at temperatures higher than 100°C which minimizes the decomposition side products. Using a solvent also allows for purification of the product. This can be advantageous not only for crystallization of crude aripiprazole but also for relatively pure aripiprazole. Thus, Type II can be directly precipitated without the need to heat treat.

The solvents used in the present invention consists of 2-propanol, dimethyl sulfoxide and combinations thereof with ethyl acetate. Generally the 2-propanol or dimethyl sulfoxide in combination with ethyl acetate comprises at least 30% by volume, more typically at least about 50% by volume, of the solution. In one embodiment, ethyl acetate is present, especially in combination with 2-propanol, and typically within the range from 1 to 75% by volume, more typically 35 to 60% by volume of the total solvent system. Surprisingly, the additional presence of ethyl acetate to the 2-propanol solvent can provide a more robust crystallization media for precipitating Type II aripiprazole than 2-propanol alone. An aripiprazole solution containing approximately a 1:1 by volume mixture of 2propanol and ethyl acetate has been found to be a useful solvent system in terms of yield and purity. In all embodiments of the present invention, the solvent(s) is/are normally anhydrous, i.e. traces of water ordinarily present in a conventional batch should be controlled and, if necessary, removed. Typically the water content within the solvent system is less than 1%.

It is surprising that the use of the above mentioned solvents in crystallization allows the formation of Type II crystals of aripiprazole. In contrast, for example, it was discovered that alcohols such as methanol and ethanol produce alcoholates, that is solvates of aripiprazole (See U.S. Provisional Application 60/628,654, filed Nov. 18, 2004). While the bound solvent can be removed by heating, no Type II was obtained by such desolvation. Instead, Form B aripirazole was formed.

The crystallization of aripiprazole as Type II from the solution as described above can be carried out by techniques generally known in the art. That is, a solution containing crude aripiprazole dissolved in the solvent system of the invention is solidified by crystallizing the dissolved aripiprazole out of the solution. The aripiprazole-containing solution can be provided in a number of ways and is not particularly limited. For example, aripiprazole can be dissolved in the solvent or it can be synthesized in the solvent.

In this regard, any form of aripiprazole may be used as the starting material for crystallization; i.e., an isolated or un-isolated crude product arisen from the synthesis of aripiprazole, which is called "crude aripiprazole" throughout this invention, or an aripiprazole product already isolated such as the Types I-III or Forms A-G as made by the techniques disclosed in the art, or an aripiprazole alcoholate such as a methanolate or a hemiethanolate as disclosed in Provisional Application 60/628,654. Typically the solvent is heated in order to increase the solubility of the crude aripiprazole. This includes forming a suspension of aripiprazole in the solvent and then heating until the solid dissolves or, alternatively, adding aripiprazole gradually into the already heated or hot solvent. A "hot" solvent has a temperature within the range of its boiling point to 20°C less than its boiling point, typically from the boiling point to 10-15°C below the boiling point of the solvent.

The concentration of aripiprazole in the solvent depends on the nature of solvent as well as the presence or absence of other dissolved or suspended components, e.g., reactants, side-products, etc. In general, the upper limit is the maximum concentration; i.e., the saturation concentration, at the boiling point of the solvent. Typically the concentration is at least about 20 to 250 mg/ml.

Once the solution containing aripiprazole has been provided, crystallization can, in general, be carried out by any convenient method. Usually the crystallization involves cooling the solution. The nucleation may be improved by adding a seeding crystal(s) or scratching the surface of the vessel. That is, the crystallization process may be induced or aided by adding small amounts of seed crystals of aripiprazole Type II.

The conditions of crystallization (concentration, cooling rate, etc.) may be controlled for the given solvent to result in the crystallization of aripiprazole Type II. With regard to 2-propanol, it is possible to form Type I or Type II, depending on the conditions. In general, higher crystallization temperatures favor Type II of the present invention while lower temperatures favor Type I as described in U.S. Provisional Application 60/628,653, filed Nov. 18, 2004. Typically for 2-propanol the crystals should initially be formed, i.e, become separated from the solution, at a temperature higher than 50°C and more typically 65°C or higher. If crystallization begins at a temperature less than 50°C, then Type I aripiprazole is generally more favored to be formed. In contrast to 2-propanol, dimethyl sulfoxide solvent apparently does not readily form Type I and thus can easily crystallize aripiprazole Type II in a more temperature independent fashion.

Other than as described above regarding the temperature concerns at the start of crystallization, the rate of cooling is not particularly limited and in general, it may affect the particle size of the formed crystals. A quicker rate of cooling generally leads to smaller crystals. A spontaneous cooling rate; i.e., allowing the solution to cool without special cooling or heating measures, as well as a linear cooling rate are generally preferred, although other cooling regimes are also contemplated for use in the present invention. The final temperature after cooling may also affect the particle size, the yield and/or the purity of the product.

As a result of crystallizing from the solvents of the invention, it is believed that aripiprazole Type II is easily and directly obtained. To confirm that the crystals are Type II, the crystals are isolated from the remaining solvent/solution and subjected to XRPD. The isolating of the crystals can be carried out by any conventional methods. In general, the solid crystalline material is recovered from the liquid portion such as by filtration or centrifugation, optionally washed such as with the solvent used or with the contrasolvent, and generally, though not necessarily, dried. The drying can be conducted *in vacuo,* with or with applying heat. It is an advantage of the process that the solvent may be removed without any long-term or high-temperature drying. The drying temperature advantageously does not exceed 60°C and preferably does not exceed 40°C. Again, it is believed that the isolated wet crystals as well as the dried crystals are aripiprazole Type II.

In further, we have discovered that aripiprazole Type II may be prepared by a solvent-mediated solid-solid transformation of a suitable metastable solid state form of aripiprazole in a liquid medium. An example of the metastable solid state form of aripiprazole is the aripiprazole Form B (as defined in WO 03/26659). The transformation may occur by suspending the starting material in the liquid medium (which is a liquid in which the aripiprazole is not soluble or only sparingly soluble) and stirring the mixture for the time necessary for completing the conversion. The degree of conversion may be monitored by suitable methods. In general, heating or cooling the suspension is not necessary, but is not excluded. Typically the temperature is within the range of 0 to 30°C such as ambient temperature. In an example, Aripiprazole Form B may be converted into aripiprazole Type II by stirring in ethyl acetate suspension at room temperature for 15 hours. It is not suggested to use water or an alcohol within the medium, as hydrates or alcoholates of the aripiprazole might be formed, which is not suitable for the purpose of this invention.

After completing the conversion, the produced Type II aripiprazole may be isolated by any convenient way of separation from the medium, e.g. by filtration or centrifugation, and optionally dried to remove the rest of the liquids

The processes of the present invention may be used for conversion of an undesired form of aripiprazole into the Type II aripiprazole, or for a purification of the insufficiently pure Type II aripiprazole. In particular, the crystallization process is well suited for this purpose as most of the impurities present in a crude starting material are well removed within the crystallization process of the present invention. The purification effect may be enhanced by using a surface active material prior to subjecting the aripiprazole solution to the crystallization; as such material may adsorb various impurities on its surface. Any conventional material, for instance activated carbon, Hyflo etc., may be used for this purpose. After treatment of the aripiprazole solution with such material, the material is normally removed such as by filtration, before carrying out crystallization. Thus, the process of the invention may be used to make essentially pure Type II of aripiprazole, i.e., essentially free from other forms of aripiprazole and/or from structurally related impurities. The essentially pure aripiprazole Type II comprises more than 98% of the Type II.

If the particle size distribution obtained as a result of the process of the invention is insufficient for the intended purpose, e.g., the dissolution profile, bioavailability of the aripiprazole, etc., is not within a desired range for the solvents dimethylsulfoxide and mixtures of 2-propanol or dimethylsulfoxide with ethyl acetate, then sieving of the crystal particles can be used to modify the population. In general, the useful population for pharmaceutical applications should contain at least 95% of particles having a particle size less than 200 microns, and in some embodiments less than 50 microns. The desired population can be preferably obtained by sieving with one or more sieves. Milling should be avoided as polymorphic transitions may occur during milling. Unlike milling, the sieving process does not bring significant energy to bear on the aripiprazole crystalline material and the crystalline form is not usual adversely affected by the process.

This surprising fact relates not only to the aripiprazole Type II prepared by the process of the present invention, but also to the Type II prepared by methods of the prior art or any other process.

Aripiprazole Type II, sieved through a sieve of the mesh size of less than 200 microns, (= a population of particles of Aripiprazole Type II, wherein more than 95% of particles has a particle size less than 200 microns, preferably between 50 and 200 microns) and also aripiprazole Type II, sieved through a sieve of the mesh size of less than 50 microns (= a population of particles of Aripiprazole Type II, wherein more than 95% of particles has a particle size less than 50 microns, preferably between 5 and 50 microns), has improved manufacturing and dissolution characteristics, particularly in making pharmaceutical tablets.

The aripiprazole Type II, (and particularly the preferred population of particles specified above) can be formulated into a pharmaceutical composition, especially a tablet or capsule, by combining the same with one or more pharmaceutically acceptable excipients. Generally the amount of aripiprazole is within the range of 1 to 50 mg per unit dose, and specially 2, 5, 10, 15, 20, 5, or 30 mg per tablet.

The present invention is more particularly described and explained by the following examples.

### EXAMPLES

### Reference Example: method to make Type II, Aoki et al

0.3 g of aripiprazole (Type I) was placed in a glass bottle of 10 ml with screw cap. The sample was annealed in an oven at 140 °C for 15 hours. The melt was slowly cooled to room temperature. Beige, opaque agglomerates of needle-like and fibre-like crystals were obtained. A small fraction of a brown glass was also present. The yield was not determined.
DSC: Irregular endothermic effects between 90-120°C. Melting peak around 147-150°C. TGA: Only gradual mass loss above 220 °C (thermal degradation). XRPD: Similar to the XRPD spectrum of Type II reported by Aoki et al. HSM: Agglomerates of plate and fibre-like crystals, mainly opalescent to nearly opaque. Between 100-125 °C there is a clear solid-solid transition (crystal jumping and cracking). All crystals melt between 145-155 °C.

### Example 1

500 mg of aripiprazole (Type I) was suspended in 10 ml of ethyl acetate at room temperature. The suspension was stirred in a closed bottle with screw cap for about 24 hours. The crystals were isolated by filtration over a P3-glass filter (reduced pressure) and air-dried overnight at ambient conditions. A white, fine powder with some lumps was obtained. The yield was 410 mg.
DSC: faint (evaporation) endotherm between 50-90°C, an asymmetric endotherm between 115-135 °C and a melting peak around 148-149 C. TGA: minimal mass loss below 80°C (some water or residual solvent). Gradual mass loss above 220°C (thermal degradation). XRPD: Corresponds to Type II. HSM: Small and irregular plates and fragments of plates.

### Example 2 (not according to the invention)

10 g of aripiprazole (Type I) was suspended in 50 ml of 2-propanol. The mixture was refluxed. To the hot suspension, 80 ml of 2-propanol was added. A clear solution was obtained. Reflux was continued for 10 minutes. The heating bath was removed, crystallisation started after 4 minutes. (T=73°C). The suspension was allowed to cool to room temperature and stirring was continued for 16 hours. The solid was isolated by filtration over a P3 glass filter and dried in a vacuum oven at 40 C for 2 hours. A white, crystalline powder with a yield of 9.42 g was obtained.

The crystals were dried at 90°C and under vacuum for an additional 16 hours and analysed.
DSC: An (asymmetric) endotherm between 95-135 °C and a melting peak around 148-149 °C.
TGA: No mass loss up to 220 °C detected.
XRPD: Corresponds to Type II.
KF: no water detected
HSM: Mainly isolated plates. The particle size of the crystals is between 10-60 mm.

### Example 3

1.01 g of aripiprazole (form B) was dissolved in 5 ml of DMSO at reflux. The hot solution was allowed to cool to room temperature. After 15 minutes, the flask was scratched with a spatula in order to induce nucleation sites. After an additional 30 minutes crystallisation could be observed. Crystallisation occurred in the next 45 minutes. Then, the crystals were isolated by filtration over a P3-glass filter (reduced pressure) and dried overweekend at 40 °C and under vacuum. Shiny and colourless to slightly bluish or greyish crystals were obtained. The yield was 180 mg.
DSC: Irregular endotherm between 95-120 °C and a large melting peak around 148-150°C.
XRPD: Corresponds to Type II.
HSM: Thick needles and plates with different crystal dimensions.

### Example 4 (not according to the invention)

40.0 g of aripiprazole (Type I) was suspended in 520 ml of 2-propanol. The mixture was stirred and heated to reflux, resulting in a clear solution. Reflux was continued for about 45 minutes. The heating bath was removed and about 80-100 mg of aripiprazole, Type II, was added as seed in 4 steps. During addition of the seed, the solution was stirred. After the fourth addition, crystallization started. The inner temperature was about 68°C. The suspension was allowed to cool to R.T. After cooling, an additional 20-40 mg of aripiprazole, Type II, was added. The suspension was overnight stirred at R.T. The solid was isolated by filtration over a P3 glass filter and dried overnight in a vacuum oven at 40 C. An off-white, crystalline powder with a greyish or bluish tinge was obtained. The yield was 38.48 g.
DSC: Corresponds to Type II.
TGA: No mass loss up to 220 °C detected.
XRPD: Corresponds to Type II.
HSM: Prism-like plates and rods, typically between 20-200 mm. Few particles > 200 mm.

### Example 5 (not according to the invention)

100 g Aripiprazole was suspended in 1.3 1 of 2-propanol. The mixture was refluxed. After 1 hour a clear solution was obtained. The suspension was allowed to cool to room temperature, wherein at 6 73°C the solution was seeded with 6 50 mg of Aripiprazol Type II crystals. Crystallization started at that temperature. Stirring was continued for 16 hours. The solid was isolated by filtration over a P3 glass filter. The solid was dried in a vacuum oven at 40° C for 24 hours. Yield: 94 g of Type II aripiprazole

### Example 6

140 g of aripiprazole was suspended in a mixture of 1 litre 2-propanol and 1 litre ethyl acetate. The stirred suspension was heated to reflux. A clear solution was obtained. Reflux was maintained for about 15 minutes. The stirred solution was allowed to cool to 55° C. At 55° C the solution was seeded with 1 g of aripiprazole Type II crystals and precipitation commenced. The seeded solution was cooled to 4° C in about 1 hour and 20 minutes. The resulting suspension was stirred at 0° -4° C for 30 minutes. The solid was isolated by filtration and dried in a vacuum oven at 40° C, <10 mbar for 16 hours. The yield was 125 g (89%) of Type II aripiprazole.

## Claims

1. A process of making crystalline aripiprazole Type II which aripiprazole Type II having a XRPD pattern shown in Figure 2, which comprises:
a) crystallizing aripiprazole Type II from a solution of aripiprazole dissolved in a solvent selected from the group consisting of dimethyl sulfoxide, and mixtures of 2-propanol or dimethyl sulfoxide with ethyl acetate, or
b) crystallizing aripiprazole Type II from a solution of aripiprazole dissolved in a solvent consisting of 2-propanol which further comprises sieving said crystalline aripiprazole Type II to obtain a desired particle size distribution, wherein said desired particle size distribution has an average particle size of less than 200 microns, preferably less than 50 microns..

2. The process according to 1, wherein the solvent has a water content of less than 1%.

3. The process according to claim 1 or 2, wherein said solvent is a mixture of 2-propanol with ethyl acetate.

4. The process according to claim 1-3, wherein 2-propanol and ethyl acetate is present in an amount of 1:1 by volume.

5. The process according to claim 1-4, which further comprises isolating said crystalline aripiprazole Type II from said solvent.

6. The process according to claim 5, wherein said isolating comprises filtering and/or drying.

7. The process according to claim 1-6, wherein said aripiprazole solution is provided as part of a synthesizing route to make aripiprazole.

8. The process according to claim 7, wherein said providing step comprises dissolving aripiprazole in said solvent to form said solution.

9. The process according to claim 7or 8, wherein said solvent is 2-propanol.

10. The process according to claim 7-9, wherein said solution further comprises ethyl acetate.

11. The process according to claim 10, wherein said ethyl acetate is present in an amount of 1:1 by volume with said 2-propanol.

12. The process according to claim 1-11, wherein said crystallizing comprises cooling said solution.

13. The process according to claim 1-12, wherein said crystallizing comprises adding a seeding crystal of aripiprazole Type II.

14. The process according to claim 1-13, wherein said recovering step comprises separating said crystals from said solvent by filtration and drying said separated crystals.

15. The process according to claim 14, wherein said drying is carried out at a temperature of 40°C or less.

16. A solvent-mediated solid-solid conversion process for making aripiprazole Type II having a XRPD pattern shown in Figure 2, which comprises stirring a suspension of aripiprazole Form B in ethyl acetate for a sufficient time to obtain conversion to aripiprazole Type II.

17. The process according to claim 16, wherein said stirring is carried out at a temperature within the range of 0 to 30°C.

18. The process according to claims 1-17 which further comprises sieving said crystalline aripiprazole Type II to obtain a desired particle size distribution.

19. The process according to claim 18, wherein said desired particle size distribution has an average particle size of less than 200 microns, preferably less than 50 microns.

20. The process according to claim 18-19, wherein said sieving comprises removing large particles by passing said crystalline aripiprazole Type II through a sieve

21. Use of a solvent selected from the group consisting of dimethyl sulfoxide, and mixtures of 2-propanol or dimethyl sulfoxide with ethyl acetate in making crystalline aripiprazole Type II having a XRPD pattern shown in Figure 2.

## Patentansprüche

1. Verfahren zur Herstellung von kristallinem Aripiprazol Typ II, wobei das Aripiprazol Typ II ein in Figur 2 gezeigtes XRPD-Muster aufweist, umfassend:
a) Kristallisieren von Aripiprazol Typ II aus einer Lösung von Aripiprazol, gelöst in einem Lösungsmittel, ausgewählt aus der Gruppe bestehend aus Dimethylsulfoxid und Mischungen von 2-Propanol oder Dimethylsulfoxid mit Ethylacetat, oder
b) Kristallisieren von Aripiprazol Typ II aus einer Lösung von Aripiprazol, das in einem Lösungsmittel, bestehend aus 2-Propanol, gelöst ist, ferner umfassend das Sieben des kristallinen Aripiprazol Typ II, um eine gewünschte Teilchengrößenverteilung zu erhalten, wobei die gewünschte Teilchengrößenverteilung eine durchschnittliche Teilchengröße von weniger als 200 Mikrometer, bevorzugt weniger als 50 Mikrometer, aufweist.

2. Verfahren nach Anspruch 1, wobei das Lösungsmittel einen Wassergehalt von weniger als 1 % aufweist.

3. Verfahren nach Anspruch 1 oder 2, wobei das Lösungsmittel eine Mischung aus 2-Propanol mit Ethylacetat ist.

4. Verfahren nach Anspruch 1-3, wobei 2-Propanol und Ethylacetat in einer Menge von 1:1 nach Volumen vorhanden sind.

5. Verfahren nach Anspruch 1-4, ferner umfassend das Isolieren des kristallinen Aripiprazol Typ II aus dem Lösungsmittel.

6. Verfahren nach Anspruch 5, wobei das Isolieren Filtrieren und/oder Trocknen umfasst.

7. Verfahren nach Anspruch 1-6, wobei die Aripiprazollösung als Teil einer Syntheseroute zur Herstellung von Aripiprazol bereitgestellt wird.

8. Verfahren nach Anspruch 7, wobei der Bereitstellungsschritt das Lösen von Aripiprazol in dem Lösungsmittel zum Bilden der Lösung umfasst.

9. Verfahren nach Anspruch 7 oder 8, wobei das Lösungsmittel 2-Propanol ist.

10. Verfahren nach Anspruch 7-9, wobei die Lösung ferner Ethylacetat umfasst.

11. Verfahren nach Anspruch 10, wobei das Ethylacetat in einer Menge von 1:1 nach Volumen mit dem 2-Propanol vorhanden ist.

12. Verfahren nach Anspruch 1-11, wobei das Kristallisieren das Kühlen der Lösung umfasst.

13. Verfahren nach Anspruch 1-12, wobei das Kristallisieren die Zugabe eines Impfkristalls von Aripiprazol Typ II umfasst.

14. Verfahren nach Anspruch 1-13, wobei der Rückgewinnungsschritt das Abtrennen der Kristalle aus dem Lösungsmittel durch Filtrieren und Trocknen der abgetrennten Kristalle umfasst.

15. Verfahren nach Anspruch 14, wobei das Trocknen bei einer Temperatur von 40 °C oder weniger durchgeführt wird.

16. Lösungsmittel-vermitteltes Feststoff-Feststoff-Umwandlungsverfahren zur Herstellung von Aripiprazol Typ II mit dem in Figur 2 gezeigten XRPD-Muster, umfassend das Rühren einer Suspension von Aripiprazol Form B in Ethylacetat für eine ausreichende Zeit, um eine Umwandlung in Aripiprazol Typ II zu erhalten.

17. Verfahren nach Anspruch 16, wobei das Rühren bei einer Temperatur in einem Bereich von 0 bis 30 °C durchgeführt wird.

18. Verfahren nach einem der Ansprüche 1-17, ferner umfassend das Sieben des kristallinen Aripiprazol Typ II, um eine gewünschte Teilchengrößenverteilung zu erhalten.

19. Verfahren nach Anspruch 18, wobei die gewünschte Teilchengrößenverteilung eine durchschnittliche Teilchengröße von weniger als 200 Mikrometer, bevorzugt weniger als 50 Mikrometer, aufweist.

20. Verfahren nach Anspruch 18-19, wobei das Sieben das Entfernen großer Teilchen umfasst, indem das kristalline Aripiprazol Typ II durch ein Sieb geleitet wird.

21. Verwendung eines Lösungsmittels, ausgewählt aus der Gruppe bestehend aus Dimethylsulfoxid und Mischungen von 2-Propanol oder Dimethylsulfoxid mit Ethylacetat, zur Herstellung von kristallinem Aripiprazol Typ II, das ein in Figur 2 gezeigtes XRPD-Muster aufweist.

## Revendications

1. Procédé de préparation d'aripiprazole cristallin de Type II, lequel aripiprazole de Type II ayant un diagramme de diffraction de rayons X sur poudre représenté dans la figure 2, qui comprend :
a) la cristallisation d'aripiprazole de Type II à partir d'une solution d'aripiprazole dissoute dans un solvant choisi dans le groupe constitué du diméthylsulfoxyde et de mélanges de 2-propanol ou de diméthylsulfoxyde avec de l'acétate d'éthyle, ou
b) la cristallisation d'aripiprazole de Type II à partir d'une solution d'aripiprazole dissoute dans un solvant constitué de 2-propanol qui comprend en outre le tamisage dudit aripiprazole cristallin de Type II pour obtenir une distribution granulométrique souhaitée, dans lequel ladite distribution granulométrique souhaitée a une taille moyenne de particules inférieure à 200 microns, de préférence inférieure à 50 microns.

2. Procédé selon la revendication 1, dans lequel le solvant a une teneur en eau inférieure à 1 %.

3. Procédé selon la revendication 1 ou 2, dans lequel ledit solvant est un mélange de 2-propanol avec de l'acétate d'éthyle.

4. Procédé selon les revendications 1-3, dans lequel le 2-propanol et l'acétate d'éthyle sont présents dans un rapport de 1:1 en volume.

5. Procédé selon les revendications 1-4, qui comprend en outre l'isolement dudit aripiprazole cristallin de Type II dudit solvant.

6. Procédé selon la revendication 5, dans lequel ledit isolement comprend la filtration et/ou le séchage.

7. Procédé selon les revendications 1-6, dans lequel ladite solution d'aripiprazole est fournie dans le cadre d'une voie de synthèse pour préparer de l'aripiprazole.

8. Procédé selon la revendication 7, dans lequel ladite étape de fourniture comprend la dissolution d'aripiprazole dans ledit solvant pour former ladite solution.

9. Procédé selon la revendication 7 ou 8, dans lequel ledit solvant est le 2-propanol.

10. Procédé selon les revendications 7-9, dans lequel ladite solution comprend en outre de l'acétate d'éthyle.

11. Procédé selon la revendication 10, dans lequel ledit acétate d'éthyle est présent dans un rapport de 1:1 en volume avec ledit 2-propanol.

12. Procédé selon les revendications 1-11, dans lequel ladite cristallisation comprend le refroidissement de ladite solution.

13. Procédé selon les revendications 1-12, dans lequel ladite cristallisation comprend l'ajout d'un cristal germe d'aripiprazole de Type II.

14. Procédé selon les revendications 1-13, dans lequel ladite étape de récupération comprend la séparation desdits cristaux dudit solvant par filtration et le séchage desdits cristaux séparés.

15. Procédé selon la revendication 14, dans lequel ledit séchage est effectué à une température de 40 °C ou moins.

16. Procédé de conversion solide-solide au moyen d'un solvant pour préparer de l'aripiprazole de Type II ayant un diagramme de diffraction de rayons X sur poudre représenté dans la figure 2, qui comprend l'agitation d'une suspension d'aripiprazole de Forme B dans de l'acétate d'éthyle pendant un temps suffisant pour obtenir une conversion en aripiprazole de Type II.

17. Procédé selon la revendication 16, dans lequel ladite agitation est effectuée à une température dans la plage de 0 à 30 °C.

18. Procédé selon les revendications 1-17, qui comprend en outre le tamisage dudit aripiprazole cristallin de Type II pour obtenir une distribution granulométrique souhaitée.

19. Procédé selon la revendication 18, dans lequel ladite distribution granulométrique souhaitée a une taille moyenne de particule inférieure à 200 microns, de préférence inférieure à 50 microns.

20. Procédé selon les revendications 18-19, dans lequel ledit tamisage comprend l'élimination de grosses particules en faisant passer ledit aripiprazole cristallin de Type II à travers un tamis.

21. Utilisation d'un solvant choisi dans le groupe constitué du diméthylsulfoxyde et de mélanges de 2-propanol ou de diméthylsulfoxyde avec de l'acétate d'éthyle dans la préparation d'aripiprazole cristallin de Type II ayant un diagramme de diffraction de rayons X sur poudre représenté sur la figure 2.
